# FASCICULE DE BREVET EUROPEEN

(11) **EP 1 663 263 B1**
(45) Date de publication et mention de la délivrance du brevet: **23.11.2011**
(21) Numéro de dépôt: 04787313.8
(22) Date de dépôt: 06.09.2004
(51) Int. Cl.: A61K 35/12, A61P 9/10

(54) **UTILISATION DE CELLULES ISSUES DU TISSU ADIPEUX POUR INDUIRE LA FORMATION D'UN RESEAU VASCULAIRE FONCTIONNEL.**
VERWENDUNG VON FETTGEWEBEZELLEN ZUR EINLEITUNG DER BILDUNG EINES FUNKTIONALEN GEFÄSSNETZES
USE OF ADIPOSE TISSUE CELLS FOR INITIATING THE FORMATION OF A FUNCTIONAL VASCULAR NETWORK

(30) Priorité: 05.09.2003 FR 0310504
(43) Date de publication de la demande: 07.06.2006
(73) Titulaire: CENTRE NATIONAL DE LA RECHERCHE SCIENTIFIQUE (CNRS), 75794 Paris Cedex 16 (FR); INSTITUT NATIONAL DE LA SANTE ET DE LA RECHERCHE MEDICALE (INSERM), 75654 Paris Cédex 13 (FR); UNIVERSITE PAUL SABATIER (TOULOUSE III) Etablissement public à caractère scientifique, culturel et professionnel, 31062 Toulouse Cedex 4 (FR); UNIVERSITE PARIS VII, F-75251 Paris Cedex 05 (FR)
(72) Inventeur: CASTEILLA, Louis, F-31750 Escalquens (FR); SILVESTRE, Jean-Sébastien, F-75012 Paris (FR); PLANAT-BENARD, Valérie, F-31120 Lacroix Falgarde (FR); LEVY, Bernard, F-75009 Paris (FR); PENICAUD, Luc, F-31400 Toulouse (FR); TEDGUI, Alain, F-75001 Paris (FR)
(74) Mandataire: Vialle-Presles, Marie José
(86) Numéro de dépôt international: PCT/FR2004/002258
(87) Numéro de publication internationale: WO 2005/025584

(56) Documents cités:
- FR-A- 2 819 265
- US-A1- 2003 082 152
- REHMAN JALEES ET AL: "Angiogenic potential of subcutaneous adipose stromal cells for autologous cell therapy." JOURNAL OF THE AMERICAN COLLEGE OF CARDIOLOGY, vol. 41, no. 6 Supplement A, 19 mars 2003 (2003-03-19), page 308A, XP009028844 52nd Annual Scientific Session of the American College of Cardiology;Chicago, IL, USA; March 30-April 02, 2003 ISSN: 0735-1097 (ISSN print)
- BOULOUMIE A ET AL: "Angiogenesis in adipose tissue" ANNALES D'ENDOCRINOLOGIE, vol. 63, no. 2 Cahier 1, 2002, pages 91-95, XP009028857 ISSN: 0003-4266
- GIMBLE JEFFREY M: "Adipose tissue-derived therapeutics." EXPERT OPINION ON BIOLOGICAL THERAPY. AUG 2003, vol. 3, no. 5, août 2003 (2003-08), pages 705-713, XP009044158 ISSN: 1471-2598
- WANG X ET AL: "Nutritional regulation of white adipocyte vascular endothelial growth factor (VEGF)" HORMONE AND METABOLIC RESEARCH, THIEME-STRATTON, STUTTGART, DE, vol. 35, no. 4, avril 2003 (2003-04), pages 211-216, XP009028856 ISSN: 0018-5043
- GRONTHOS S ET AL: "Surface protein characterization of human adipose tissue-derived stromal cells", JOURNAL OF CELLULAR PHYSIOLOGY, vol. 189, 2001, pages 54-63,

## Description

La présente invention est relative à l'utilisation de cellules issues du tissu adipeux blanc médullaire ou extra-médullaire et notamment de la fraction stromale vasculaire extra-médullaire (FSV) et/ou d'adipocytes matures dédifférenciés quelle qu'en soit l'origine, pour induire la formation d'une vascularisation fonctionnelle.

Il existe un besoin, notamment dans les sociétés occidentales, de moyens thérapeutiques efficaces stimulant la néovascularisation, pour limiter les complications associées aux pathologies ischémiques et/ou favoriser la régénération tissulaire.

Jusqu'à présent, les stratégies thérapeutiques proposées pour limiter les effets délétères de l'ischémie font appel à la stimulation de la croissance et du remodelage des vaisseaux sur le lieu même de l'ischémie et/ou à la transplantation de cellules progénitrices endothéliales. Pour l'obtention de cellules endothéliales aptes à permettre effectivement une revascularisation d'un secteur ischémié, les méthodes proposées jusqu'à présent sont essentiellement basées sur l'obtention de cellules endothéliales matures à partir de cellules progénitrices endothéliales circulantes de l'adulte. Ces cellules mononucléées humaines d'origine hématopoïétique, issues de la lignée monocytes/macrophages (CD45+, CD14+) sont isolées à partir de la moelle osseuse (BM-MNC pour *bone marrow-mononuclear cells*) ou du sang périphérique, en présence de facteurs de croissance angiogéniques (20 ; 21 ; 27 ; 38 ; 39).

Plus particulièrement, il a été montré que la transplantation de BM-MNC (*bone marrow-mollonuclear cells*) stimulait effectivement la néovascularisation dans des ischémies expérimentales et entraînait une amélioration significative et une survie à long terme des tissus lésés (26 ; 27). Les essais effectués chez l'homme montrent le potentiel d'une telle thérapie pour limiter la progression de la maladie (28-32).

Toutefois, le faible pourcentage et la difficulté d'expansion *ex vivo* de ces cellules progénitrices endothéliales et la détérioration fonctionnelle de ces cellules, observée dans des conditions pathologiques, constituent une limitation majeure à leur utilisation dans le traitement de l'ischémie.

En conséquence, il existe donc un réel besoin de disposer d'une source de cellules formant une population cellulaire homogène, capable de se différencier en cellules endothéliales matures, utilisables notamment dans le cadre de la réparation de tissus lésés par ischémie, qui soit simple à obtenir et efficace.

Le tissu adipeux existe sous différentes formes chez les mammifères : le tissu adipeux blanc extramédullaire qui représente le principal organe de réserve de l'organisme, le tissu adipeux blanc médullaire dont le rôle exact n'est pas connu et le tissu adipeux brun thermogénique.

Du fait de son potentiel d'expansion important qui persiste tout au long de la vie de l'individu, le tissu adipeux blanc de l'adulte constitue une source de cellules abondantes et faciles à obtenir.

Ce tissu adipeux blanc est constitué par deux fractions cellulaires :
- une fraction adipocytaire qui représente 30 % à 60 % des cellules du tissu adipeux et est caractérisée par l'accumulation de triglycérides (fraction de cellules flottantes). Cette fraction est composeé en grande majorité (99 %) d'adipocytes différenciés et de quelques macrophages contaminants, riches en goutelettes lipidiques, et
- une fraction non-adipocytaire, appelée fraction du stromavasculaire (FSV) comprenant quelques cellules sanguines, quelques cellules endothéliales matures (cellules de l'endothélium microvasculaire : CD31⁺, CD144⁺), des péricytes, des fibroblastes et des cellules souches pluripotentes.

Il a été montré que la fraction stromale vasculaire, classiquement utilisée pour étudier la différenciation des préadipocytes en adipocytes matures, était une source de cellules souches pluripotentes comprenant outre des progéniteurs adipocytaires (préadipocytes), uniquement des progéniteurs hématopoïétiques et neurogéniques, ainsi que des cellules souches mésenchymateuses capables de se différencier en lignées ostéogéniques, chondrogéniques et myogéniques (10 ; 11 ; 12 ; 13 ; Demande Internationale PCT WO 02/055678 et Demande américaine US 2003/0082152).

Ces deux fractions cellulaires peuvent être séparées par leur différence de densité, selon des procédés tels que ceux décrits par Bjôrntorp et al. (14).

Le tissu adipeux blanc possède des propriétés angiogéniques uniques résultant de l'effet sur les cellules endothéliales vasculaires différenciées, de facteurs pro-angiogéniques produits par les adipocytes (Bouloumié et al., Ann. Endocrin., 2002, 63, 91-95 ; Wang et al., Horm. Metab. Res., 2003, 35, 211-216) et les cellules de la fraction stromale vasculaire (Rehman et al., Journal of the American College of Cardiology, 2003, 41, 6 supplément A, 308A). Ces propriétés angiogéniques qui jouent vraisemblablement un rôle dans l'activité métabolique et l'expansion du tissu adipeux ont des applications en thérapie cellulaire autologue, pour favoriser l'angiogénèse dans un contexte post-traumatique ou pathologique (post-ischémique). Ainsi, l'injection de tissu adipeux autologue est couramment pratiquée en chirurgie, pour favoriser la re-vascularisation des greffes et la reconstruction des tissus mous (Bouloumié et al. ; Wang et al., précités). En outre, il a également été préconisé d'administrer la fraction stromale vasculaire autologue, pour favoriser l'angiogénèse, dans le traitement de la maladie coronarienne (Rehman et al., précité).

Toutefois, la néo-vascularisation résulte non-seulement de l'effet de facteurs pro-angiogéniques sur les cellules endothéliales des vaisseaux préexistants (angiogénèse) mais aussi de la production et de l'incorporation dans les vaisseaux en formation, de cellules endothéliales différenciées, produites à partir de cellules progénitrices endothéliales (vasculogénèse).

De telles cellules progénitrices endothéliales, n'ont pas été isolées à partir du tissu adipeux et notamment de la fraction stromale vasculaire contenant des cellules pluripotentes.

Dans ce contexte, les Inventeurs ont isolé une sous-population homogène de cellules du tissu adipeux blanc médullaire ou extra-médullaire (faciles à obtenir (liposuccion, par exemple), capable de se différencier en cellules endothéliales matures qui permettent d'obtenir une reconstruction totale ou partielle d'un réseau vasculaire fonctionnel.

La présente invention a en conséquence pour objet l'utilisation de cellules du tissu adipeux blanc médullaire ou extra-médullaire formant des sous-populations homogènes, exprimant au moins les antigènes de surface CD13 et HLA ABC et CD34 (CD13⁺, HLA ABC⁺, CD34⁺ ) et ne pas exprimant les antigènes de surface CD14, CD45, CD31 et CD144, pour la préparation d'un médicament destiné à la reconstruction totale ou partielle d'un réseau vasculaire fonctionnel, notamment dans le contexte d'une ischémie.

Selon un second mode de réalisation avantageux de ladite utilisation, lesdites cellules du tissu adipeux sont représentées par une sous-population homogène de cellules de la fraction stromale vasculaire extra-médullaire (dénommées ci-après FSV-CULT), susceptible d'être obtenue par expansion cellulaire limitée en culture.

Selon une disposition avantageuse de ce mode de réalisation, ladite sous-population homogène de cellules de la fraction stromale vasculaire extra-médullaire est susceptible d'être obtenue par une expansion cellulaire limitée par un nombre de passages successifs inférieur à 10 desdites cellules.

Ainsi, de manière surprenante, une expansion cellulaire limitée, du fait du nombre de passages successifs limités à 10 au plus, favorise la prolifération d'une population homogène de cellules possédant des antigènes de surface qui sont caractéristiques des cellules à potentiel pro-angiogénique, mais qui ne possèdent aucun marqueur de surface caractéristique des cellules hématopoïétiques incluant celles de la lignée des monocytes/macrophages ou des cellules endothéliales différenciées.

Egalement de manière surprenante, on obtient de telles cellules par culture en milieu minimum, tel qu'un milieu DMEM comprenant 10 % de sérum de veau foetal ou de nouveau-né, par exemple.

Des conditions particulières, induisant plus rapidement des caractéristiques pro-angiogéniques, peuvent également être mises en oeuvre. Elles sont précisées ci-après (voir procédé de sélection de cellules du tissu adipeux).

Selon un troisième mode de réalisation avantageux de ladite utilisation, lesdites cellules du tissu adipeux sont représentées par une sous-population homogène d'adipocytes matures dédifférenciés (dénommées ci-après DDAC).

Les adipocytes dédifférenciés sont notamment obtenus dans les conditions décrites dans Negrel R. et al. (17) ou dans Shigematsu M. et al. (19).

Ainsi, par expansion limitée de la fraction stromale vasculaire extra-médullaire ou par dédifférenciation d'adipocytes matures, on obtient des sous-populations de cellules exprimant au moins les antigènes de surface précités, à savoir : CD 13, HLA ABC (CD13⁺, HLA ABC⁺). L'antigène de surface CD34 qui est présent dans les cellules fraîchement isolées peut disparaître progressivement au cours des passages successifs en culture. Ces cellules par contre, n'expriment notamment pas les antigènes de surface suivants : CD45, CD 14, CD31 et CD 144 (CD45⁻, CD 14⁻, CD31⁻ et CD144⁻). Les sous-populations de cellules exprimant au moins les antigènes de surface précités sont capables de se différencier en cellules endothéliales fonctionnelles exprimant les antigènes de surface CD31 et CD 144.

Selon un quatrième mode de réalisation avantageux de ladite utilisation, lesdites cellules du tissu adipeux formant des sous-populations homogènes, exprimant au moins les antigènes de surface tels que dans la revendication 1. sont associées à un support polymérique solide ou semi-solide.

Selon une disposition avantageuse de ce mode de réalisation, ledit support polymérique solide est de préférence une matrice membranaire basale reconstituée comprenant au moins l'un des éléments suivants : collagène, laminine et protéoglycanes ou une matrice extracellulaire reconstituée comprenant l'un des éléments suivants : fibronectine, collagène, laminine et thrombospondine. Ledit support peut en outre comprendre des enzymes de dégradation desdites matrices ainsi que des inhibiteurs enzymatiques et des facteurs de croissance. On peut citer, à titre d'exemple, comme matrices particulièrement appropriées, les matrices Matrigel^{®} (Becton Dickinson ; 40).

Selon une autre disposition avantageuse de ce mode de réalisation, ledit support polymérique semi-solide est de préférence un dérivé cellulosique et notamment de la méthyl-cellulose.

Conformément à l'invention, lesdites cellules peuvent en outre être génétiquement modifiées. Ainsi :
- elles peuvent comprendre au moins une mutation d'un gène autologue ou
- elles peuvent contenir au moins une copie d'un gène hétérologue.

Lesdites cellules génétiquement modifiées sont, de préférence, d'origine humaine.

La présente invention a également pour objet l'utilisation d'une composition contenant des cellules du tissu adipeux blanc, médullaire ou extramédullaire formant des sous-populations homogènes, exprimant au moins les antigènes de surface suivants : CD13⁺, CD34⁺, HLA ABC⁺ telles que définies ci-dessus, et au moins un véhicule et/ou un support approprié à une administration parentérale ou intra-site (*in situ* au niveau de l'organe lésé), pour la préparation d'un médicament destiné à la reconstruction totale ou partielle d'un réseau vasculaire fonctionnel.

La présente invention a également pour objet une composition pharmaceutique contenant des cellules du tissu adipeux blanc, médullaire ou extramédullaire formant des sous-populations homogènes, exprimant au moins les antigènes de surface CD13 et HLA ABC telles que définies ci-dessus, lesdites cellules étant associées à un support polymérique solide ou semi-solide tel que défini ci-dessus, et au moins un véhicule et/ou un support approprié à une administration parentérale ou intrasite.

Les cellules telles que définies dans la présente invention sont utiles pour le traitement de n'importe quelle pathologie ischémique, notamment les pathologies cadiovasculaires, telles que l'athérosclérose. En effet, le facteur déclenchant l'ischémie chez un patient artéritique est la rupture d'une plaque d'athérome et la formation d'un thrombus.

Ces cellules sont actives quelle que soit la nature du tissu ischémié et peuvent être utilisées pour le traitement d'une ischémie affectant un tissu tel que notamment le cerveau, le pancréas, le foie, le muscle et le coeur.

Ces cellules sont actives quelle que soit la voie d'administration ; elles peuvent être administrées, notamment par voie générale (intramusculaire, intrapéritonéale ou intraveineuse) ou directement au niveau du tissu lésé.

La présente invention a, en outre, pour objet un procédé de culture de cellules du tissu adipeux blanc médullaire ou extra-médullaire formant des sous-populations homogènes, exprimant au moins les antigènes de surface CD13 et HLA ABC, lequel procédé est caractérisé en ce qu'il comprend au moins les étapes suivantes :
- expansion cellulaire limitée de cellules dudit tissu adipeux (cellules de la fraction stromale vasculaire extra-médullaire ou adipocytes matures dédifférenciés), par un nombre de passages successifs inférieurs à 10 desdites cellules, sur un support de culture solide convenable, dans un milieu comprenant au moins un facteur de croissance apte à stimuler la formation de cellules endothéliales et éventuellement au moins une cytokine convenable ;
- modification, de manière continue ou transitoire, de l'environnement en oxygène de la culture et
- modification de manière continue ou transitoire, de l'équilibre redox desdites cellules ou de la production d'espèces actives de l'oxygène par lesdites cellules, par ajout de molécules pro- ou antioxydantes dans le milieu extra ou intracellulaire.

Conformément à l'invention :
- lesdites cellules du tissu adipeux blanc médullaire ou extramédullaire formant des sous-populations homogènes, exprimant au moins les antigènes de surface CD13 et HLA ABC sont constituées de la fraction stromale vasculaire extra-médullaire ou d'adipocytes dédifférenciés, et
ledit milieu de culture est de préférence un milieu de culture liquide.

Selon un mode de mise en oeuvre avantageux dudit procédé, le facteur de croissance apte à stimuler la formation de cellules endothéliales est notamment du VEGF, de préférence à une concentration d'environ 10 ng/ml.

Selon un autre mode de mise en oeuvre avantageux dudit procédé, l'environnement en oxygène de la culture est à 1 % ; de quelques heures à quelques jours.

Les molécules pro- ou antioxydantes sont notamment sélectionnées dans le groupe constitué par :
- les inhibiteurs et/ou activateurs de la fonction mitochondriale et notamment l'antimycine, de préférence à une concentration comprise entre 1 et 1000 nM, de préférence 1 à 100 nM, la roténone à une concentration comprise entre 1 et 100 nM, l'oligomycine à une concentration comprise entre quelques ng et quelques µg/ml, le coenzyme Q, des nucléotides ou tout autre molécule équivalente et le carbonyl cyanide m-chlorophénylhydrazone), et
- des antioxydants sélectionnés dans le groupe constitué par le trolox, le dithiocarbamate de pyrrolidine, la N-acétyl cystéine, le manganèse (III) tetrakis (4-acide benzoïque) porphyrine ou tout autre molécule équivalente.

La présente invention a également pour objet, un procédé de criblage de molécules actives sur les cellules endothéliales différenciées, lequel procédé est caractérisé en ce qu'il comprend au moins les étapes suivantes :
- la culture de cellules du tissu adipeux blanc médullaire ou extramédullaire formant des sous-populations homogènes, exprimant au moins les antigènes de surface CD13 et HLA ABC, telles que définies ci-dessus, dans un milieu de culture polymérique semi-solide,
- la mise en contact des cellules endothéliales différenciées ainsi obtenues, avec une banque de molécules à tester,
- l'identification et la sélection des molécules actives sur lesdites cellules.

Le procédé selon l'invention est utile aussi bien pour cribler de nouvelles molécules chimiques que le produit de nouveaux gènes potentiellement actifs sur les cellules endothéliales différenciées.

Selon un mode de mise en oeuvre avantageux dudit procédé de criblage, l'étape de culture en milieu solide est précédée d'une préculture dans les conditions permettant d'augmenter le potentiel pro-angiogénique desdites cellules, telles que définies ci-dessus.

Selon un autre mode de mise en oeuvre avantageux dudit procédé de criblage, l'étape de culture en milieu semi-solide est réalisée dans des conditions permettant d'augmenter le potentiel pro-angiogénique desdites cellules, telles que définies ci-dessus.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

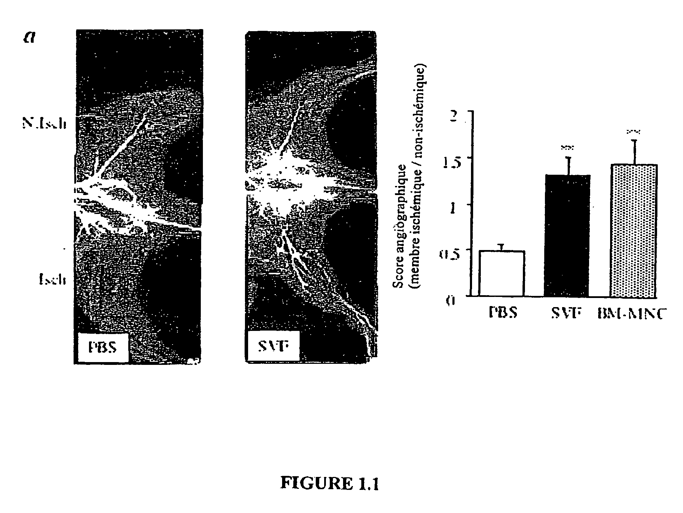

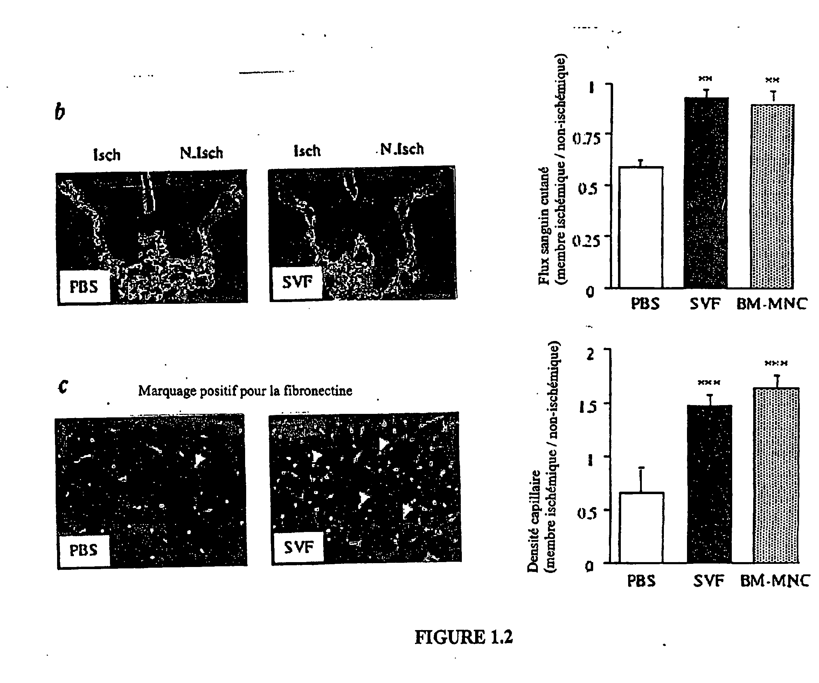

- la Figure 1 illustre les propriétés angiogéniques des cellules de souris de la fraction stromale vasculaire extra-médullaire (SVF pour *Stromal-Vascular Fraction*) cultivée dans les conditions de l'invention, après leur injection dans le membre inférieur ischémié, en comparaison avec les cellules mononucléées de la moelle osseuse (BM-MNC pour *Bone-Marrow Mononuclear cells*) ; pour plus de clarté, les cellules obtenues par culture de cette fraction dans les conditions de l'invention sont dénommées cellules FSV-CULT, dans la suite des exemples.
   a) analyse de la densité en vaisseaux par microangiographie. Panneau de gauche : microangiographie représentative d'un membre inférieur droit ischémié (Isch) et d'un membre inférieur gauche non-ischémié (N-Isch), 15 jours après occlusion fémorale. Les flèches indiquent les extrémités ligaturées de l'artère fémorale. Panneau de droite : score angiogaphique dans le membre ischémié traité, par rapport au membre non-ischémié.
   b) analyse *in vivo* du flux sanguin dans le membre inférieur par imagerie de perfusion par laser à effet Doppler, 15 jours après occlusion de l'artère fémorale. Panneau de gauche: Image du flux sanguin indiquant une perfusion normale (représentée en noir), dans le membre non-ischémie et le membre ischémié traité par les cellules FSV-CULT, ainsi qu'une nette réduction du flux sanguin dans le membre inférieur ischémié traité par le PBS. Panneau de droite : mesure du flux sanguin dans le membre ischémié traité, par rapport au membre non-ischémié.
   c) analyse de la densité capillaire par immunomarquage de la fibronectine totale. Panneau de droite : photomicrogaphies représentatives de sections de muscle ischémié, 15 jours après l'occlusion fémorale. Les capillaires, indiqués par des flèches apparaissent en blanc et les myocytes en noir. Panneau de droite : mesure de la densité capillaire dans le membre ischémié traité, par rapport au membre non-ischémié.
      PBS : Souris traitées avec du PBS. SVF : Souris traitées avec les cellules FSV-CULT. BM-MNC : Souris traitées avec des cellules de la moelle osseuse. Les valeurs représentent la moyenne ± écart-type, n=6 par groupe ; **P<0,01 ;

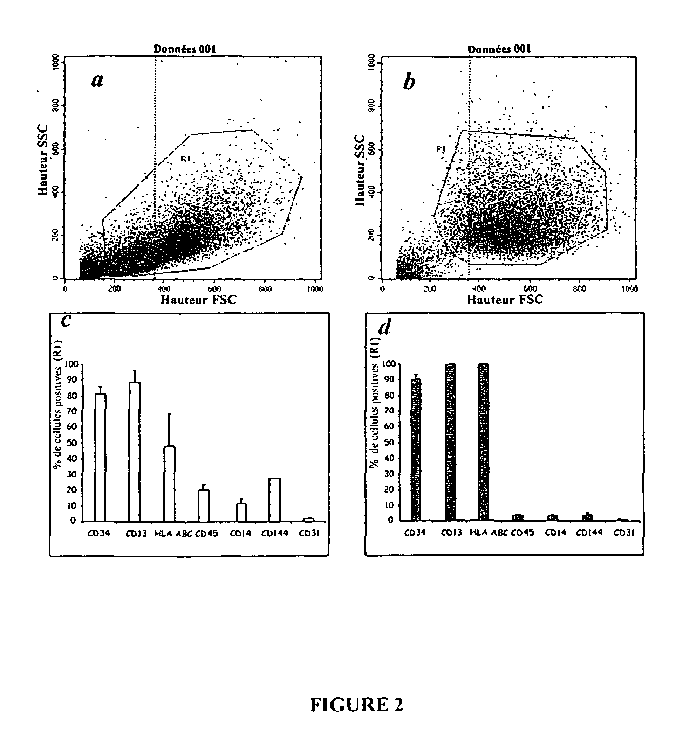

- la Figure 2 montre que l'expansion d'une population hétérogène de cellules humaines de la fraction stromale vasculaire (préparation extemporanée obtenue avant culture, dénommée ci-après FSV-EXT) dans les conditions de l'invention, favorise effectivement l'apparition d'une population cellulaire homogène (FSV-CULT) :
   a) et b) : diagrammes de dispersion des cellules FSV-EXT et des cellules FSV-CULT ; ces diagrammes comportent en abscisse une estimation de la taille des cellules (FSC *height : forward scatter height*) et en ordonnées la granulosité des cellules (SSC *height : side scatter height*).
   c) et d) : identification des antigènes CD45, CD14 et CD144, caractéristiques respectivement des cellules hématopoïétiques (CD45), des monocytes/macrophages (CD14) et des cellules endothéliales matures (CD144) dans une population hétérogène de cellules FSV-EXT (colonnes blanches) en comparaison avec des cellules FSV-CULT (colonnes noires) ;

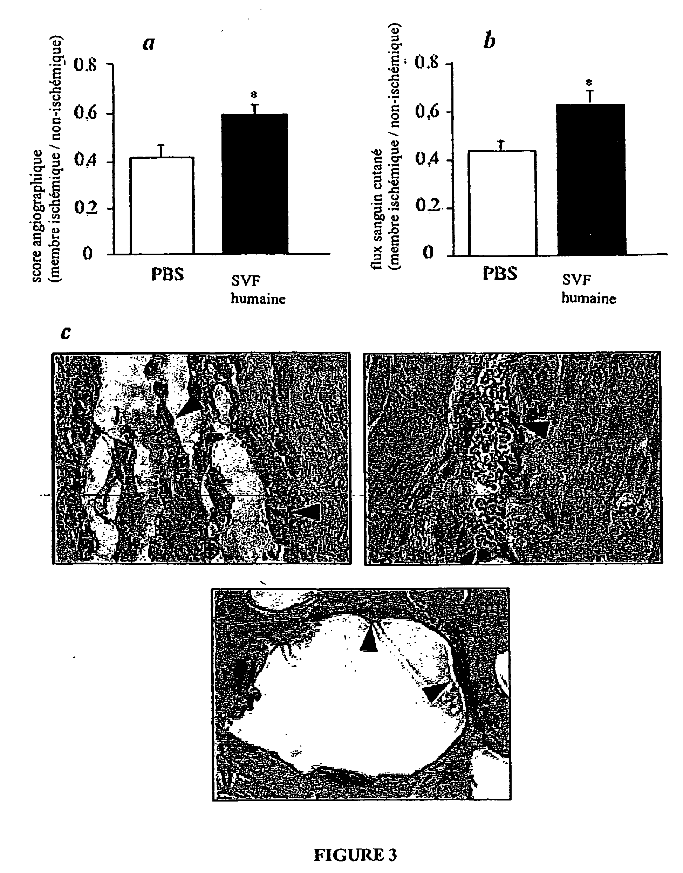

- la Figure 3 montre que les cellules humaines FSV-CULT possèdent les propriétés fonctionnelles et antigéniques de précurseurs de cellules endothéliales, après leur injection dans le membre inférieur ischémié :
   a) et b) l'injection de cellules humaines FSV-CULT augmente de façon significative le score angiographique et le flux sanguin mesuré *in vivo* par imagerie de perfusion par laser à effet Doppler, dans le membre inférieur droit ischémié greffé, lorsqu'on compare au membre inférieur gauche non-ischémié non-greffé (groupe PBS) (*P < 0,05) ;
   c) 15 jours après l'injection de cellules FSV-CULT humaines, l'anticorps dirigé spécifiquement contre une isoforme du marqueur CD31 humain, marque de nombreuses cellules CD31 positives (indiquées par des flèches noires) qui bordent des vaisseaux fonctionnels contenant des érythrocytes (indiqués par une flèche grisée à l'intérieur d'un vaisseau) (x 1000) ;

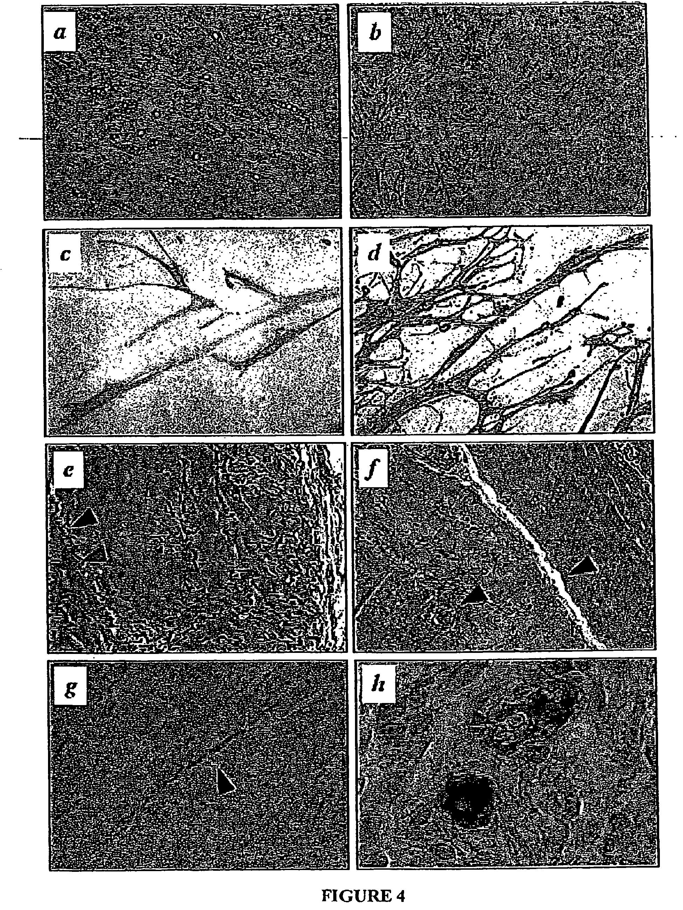

- la figure 4 illustre la différenciation des cellules FSV-CULT en cellules endothéliales dans des conditions *in vitro* ou dans une matrice de Matrigel^{®} greffée *in vivo* :
   a) différenciation des cellules FSV-CULT en adipocytes dans un milieu adipogénique (x 200) ;
   b) formation d'alignements branchus et de structures de type tubulaire, de manière spontanée lorsque les cellules FSV-CULT sont ensemencées dans un milieu contenant de la méthyl-cellulose (x 200) ;
   c) et d) marquage des cellules FSV-CULT ensemencées dans un milieu contenant de la méthyl-cellulose par des anticorps dirigés respectivement contre une isoforme du marqueur CD31 humain et contre le marqueur vWF (facteur von Willebrand) ; on note la formation d'alignements branchus (x 600) ;
   e) et f) formation dans une matrice de Matrigel^{®} contenant les cellules FSV-CULT et greffée *in vivo* de structures de type tubulaire (indiquées par des flèches noires) ; on observe également des érythrocytes dans les structures de type tubulaire (indiqués par des flèches grisées) ;
   g) et h) marquage des cellules FSV-CULT bordant les structures de type tubulaire de l'inclusion de Matrigel^{®}, par un anticorps anti-isoforme du marqueur CD31 humain (x 400 et 1000) ;

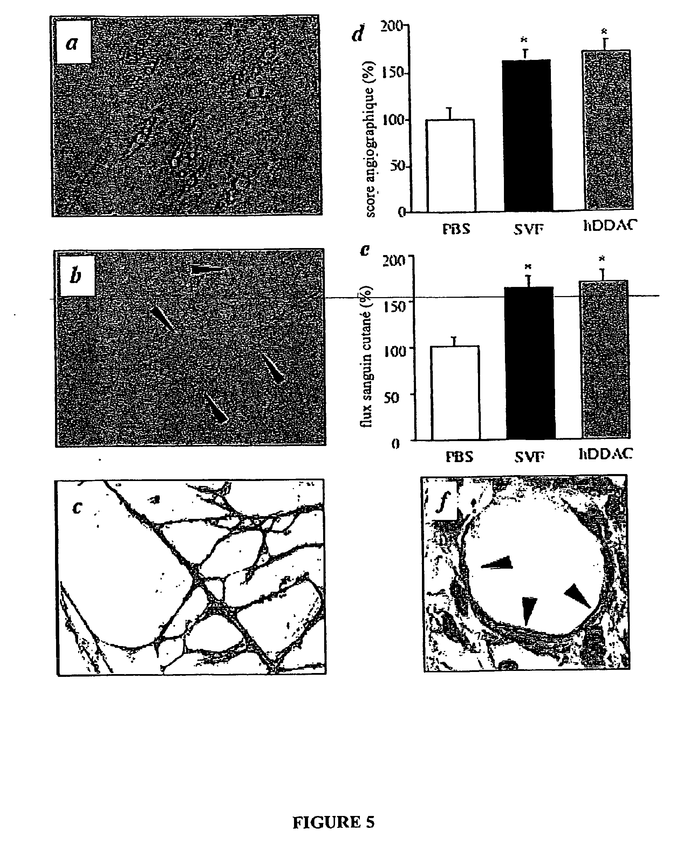

- la Figure 5 illustre la dédifférenciation des adipocytes matures en cellules progénitrices ou précurseurs à double potentiel prolifératif, qui ont la capacité d'acquérir un phénotype de cellules endothéliales :
   a) les cellules hDDAC (*human dedifferenciated adipose cells* ou cellules adipeuses humaines dédifférenciées) peuvent proliférer et se différencier à nouveau en adipocytes, lorsqu'elles sont cultivées dans un milieu adipogénique (x 400) ;
   b) les cellules hDDAC forment des alignements branchus et des structures de type tubulaire (flèches noires), lorsqu'elles sont cultivées dans un milieu comprenant de la méthyl-cellulose (x 400) ;
   c) les alignements branchus et structures de type tubulaire, formés par dédifférenciation des adipocytes matures dans un milieu de culture contenant de la méthyl-cellulose sont marqués par un anticorps anti vWF (x 400) ;
   d) et e) ces figures illustrent les propriétés pro-angiogéniques des cellules hDDAC après leur greffe dans le membre inférieur ischémié ; les cellules hDDAC sont aussi efficaces que les cellules FSV-CULT pour restaurer le score angiographique et le flux sanguin cutané dans le membre inférieur ischémié.
      Les valeurs représentent la moyenne ± écart-type, n=6 par groupe ; *P<0,05. PBS : Souris traitées avec du PBS. SVF : Souris traitées avec les cellules FSV-CULT. hDDAC : Souris traitées avec des adipocytes humains dédifférenciés ;
   f) marquage de nombreuses cellules C31 positives tapissant les vaisseaux nouvellement formés (indiqués par des flèches noires), par un anticorps dirigé contre l'isoforme du marqueur CD31 humain (x 1000) ;

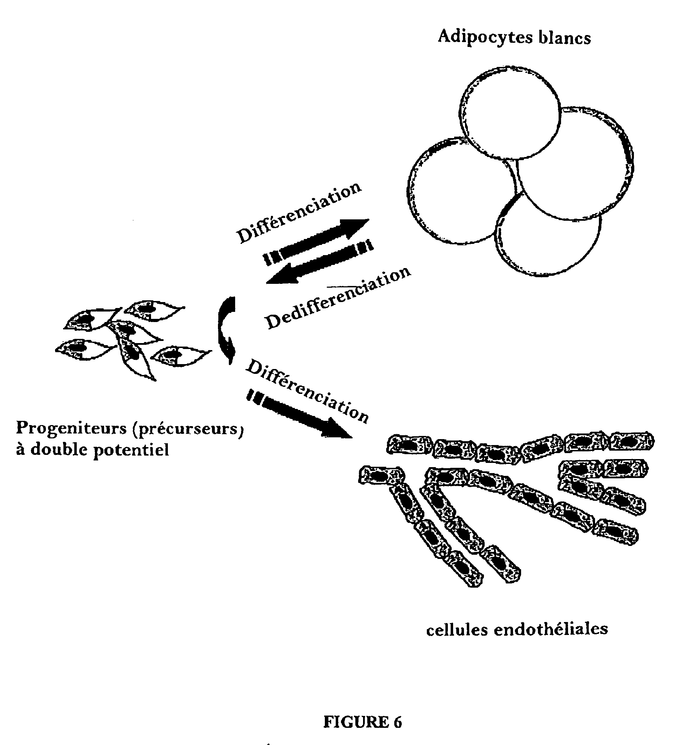

- la Figure 6 illustre la plasticité des cellules de la lignée adipocytaire, pour l'obtention de cellules endothéliales. Les cellules progénitrices adipocytaires ont la capacité de se différencier en adipocytes et d'acquérir un phénotype endothélial fonctionnel. Les adipocytes matures peuvent se dédifférencier en cellules progénitrices à double potentiel prolifératif.

Il doit être bien entendu, toutefois, que ces exemples sont donnés uniquement à titre d'illustration de l'objet de l'invention, dont ils ne constituent en aucune manière une limitation.

### EXEMPLE 1 : Induction d'une néovascularisation dans un muscle ischémié de souris à l'aide de cellules FSV-CULT de souris.

### 1.1 Matériel et méthodes

### 1.1.1 Animaux et échantillons de tissu

Des souris mâles C57B1/6 ou nu/nu (Harlan, France) âgées de 7 semaines sont élevées dans un environnement contrôlé (cycle de 12 heures de lumière et de 12 heures d'obscurité à 21°C) avec un libre accès à l'eau et à la ration alimentaire standard. A la fin des expériences, les souris sont sacrifiées par dislocation cervicale sous anesthésie au CO₂. Le tissu adipeux inguinal et le muscle sont rapidement prélevés et traités pour les analyses ultérieures.

### 1.1.2 Modèle de la souris avec un membre inférieur ischémié

Les animaux sont anesthésiés par inhalation d'isoflurane. Une ligature est réalisée sur l'artère fémorale droite. Ensuite, on leur injecte 10⁶ cellules FSV-CULT par voie intramusculaire dans le membre ischémié.

### 1.1.3 Isolement des cellules de la fraction stromale-vasculaire du tissu adipeux et des cellules de la moelle osseuse

### - Cellules de la moelle osseuse :

Les cellules de moelle osseuse sont obtenues par lavage des tibias et des fémurs puis isolement des cellules mononucléées de basse densité, par centrifugation sur gradient de densité de Ficoll (34).

### - Cellules de la fraction stromale vasculaire du tissu adipeux extramédullaire

Les cellules de la fraction stromale vasculaire sont isolées à partir de tissus adipeux selon le protocole de Bjôntorp *et al.* (14) avec des modifications mineures. Brièvement, le tissu adipeux inguinal de souris est soumis à une digestion par 2 mg/ml de collagénase (Sigma) dans du tampon phosphate PBS contenant 0,2 % de BSA à 37°C pendant 45 minutes. Après élimination des fragments non-hydrolysés par filtration sur membrane de nylon de 100 µm, les adipocytes matures sont séparés des culots de cellules FSV-EXT par centrifugation (600 g, 10 minutes).

Les cellules FSV-EXT sont ensemencées à une densité de 30 000 cellules/cm² dans du milieu DMEM F12 supplémenté par 10 % de sérum de veau nouveau-né (SVN). Après 6 heures de culture, les cellules non-adhérentes sont éliminées par lavage, puis les cellules (adhérentes) sont cultivées pendant quelques jours (1 à 3) avant d'être utilisées ; on obtient ainsi des cellules FSV-CULT.

### 1.1.4 Quantification de la néovascularisation

La densité en vaisseaux est évaluée par micro-angiographie à haute définition en fin de période de traitement (36). Le score angiographique est exprimé par le pourcentage de pixels par image occupés par des vaisseaux, dans une aire de quantification.

L'analyse micro-angiographique est complétée par l'évaluation de la densité capillaire grâce à un anticorps dirigé contre la fibronectine totale (36). La densité capillaire est alors calculée dans des champs aléatoires d'une aire définie, en utilisant le logiciel Optilab/Pro.

La fonctionalité du réseau vasculaire après l'ischémie, est analysée par imagerie de perfusion par laser à effet Doppler, réalisée chez la souris comme décrit dans Silvestre JS et al. (36).

### 1.2 Résultats

Dans un premier temps, le potentiel angiogénique du tissu adipeux a été évalué avec des cellules FSV-CULT de souris, par comparaison avec des cellules mononucléées de la moelle osseuse.

Ces cellules sont préparées à partir de tissu adipeux inguinal et mises en culture pour obtenir une expansion limitée pendant 1-3 jours (nombre de passages successifs limité à moins de 10). La greffe de 1×10⁶ cellules FSV-CULT améliore nettement la néovascularisation du tissu dans des membres inférieurs ischémiés comme le montre l'augmentation d'un facteur 2,6 du score angiographique, (Figure 1a, P<0,01), l'augmentation d'un facteur 2,3 du score de perfusion du tissu par Doppler (Figure 1b, P<0,001) et l'augmentation d'un facteur 1,6 de la densité capillaire (Figure 1c, P<0,01). Le degré de néovascularisation observé après l'injection de 1×10⁶ cellules FSV-CULT est comparable à celui observé après l'injection de 1×10⁶ cellules mononucléées de la moelle osseuse (Figures 1a-c). Le processus de culture selon l'invention améliore le potentiel angiogénique des cellules FSV-CULT de façon très significative, comme le montre la très faible néovascularisation observée après injection directe de cellules FSV-EXT (sans mise en culture avec expansion limitée comme dans l'invention). En outre, des expériences avec les cellules du stroma vasculaire provenant du tissu adipeux brun, connu pour être plus vascularisé que le tissu blanc, se sont révélées infructueuses.

### EXEMPLE 2: Caractérisation phénotypique des cellules FSV-EXT et des cellules FSV-CULT.

### 2.1 Matériel et méthodes

### 2.1.1 Préparation de cellules FSV-EXT et FSV-CULT

Les cellules FSV-EXT et FSV-CULT de souris sont préparées comme précisé dans l'Exemple 1.

Les cellules humaines correspondantes sont préparées de façon similaire, à partir d'échantillons de dermolipectomie abdominale ou de néphrectomie contenant du tissu sous-cutané abdominal humain, obtenus avec le consentement des patients.

### 2.1.2 Analyse phénotypique des cellules

Les cellules sont marquées dans du tampon salin phosphate contenant 0,2% de sérum de veau foetal ; elles sont incubées avec des anticorps monoclonaux anti-souris ou anti-humain (mAb) couplés à l'isothiocyanate de fluorescéine (FITC), à la phycoérythrine (PE) ou à la protéine de chlorophylle péridinine (PerCP) pendant 30 minutes à 4°C. Après lavage, les cellules sont analysées en cytométrie de flux (FACS Calibur, Becton Dickinson). Les données obtenues sont alors analysées en utilisant le logiciel Cell Quest (Becton Dickinson). Tous les anticorps proviennent de BD Biosciences, à l'exception de CD144 qui provient de Serotec.

### 2.1.4 Analyses statistiques

Toutes les analyses statistiques sont réalisées par t-test non-apparié en utilisant le logiciel Prisme™ (GraphPad sofware).

### 2.2 Résultats

L'analyse comparative du phénotype des cellules FSV-EXT et FSV-CULT (humaines ou murines) a été réalisée en cytométrie de flux. Les résultats obtenus avec les cellules humaines et murines étant comparables, seuls les résultats relatifs aux cellules humaines sont présentés.

Les cellules FSV-EXT obtenues à partir de tissu adipeux humain sous-cutané sont hétérogènes comme le montre le diagramme de dispersion de la Figure 2a. La culture de ces cellules pendant 1-3 jours dans les conditions de l'invention conduit à une homogénéisation de la population cellulaire, comme le montre l'obtention d'une seule population cellulaire, dénommée FSV-CULT (Figure 2b).

Le phénotype antigénique confirme le diagramme de dispersion. Les cellules FSV-EXT sont hétérogènes et comprennent différentes populations, notamment des cellules hématopoïétiques (cellules positives pour le marqueur CD45) et une population de cellules non-hématopoïétiques (négative pour le marqueur CD45), et exprimant les marqueurs CD34, CD13 et HLA ABC (Figure 2c).

La fraction stromale vasculaire ne contient pas une proportion significative de cellules endothéliales matures, comme le montre l'absence de marquage avec les anticorps dirigés contre la VE-cadhérine (CD144) et le marqueur CD31 (Figure 2c). Dans la population FSV-CULT (conditions de l'invention), la population est composée en majorité de cellules indifférenciées avec 90±3% de cellules exprimant le marqueur CD34 et 99±0,2% de cellules étant positives pour les marqueurs CD13 et HLA ABC. En revanche, ces cellules FSV-CULT n'expriment, ni les marqueurs caractéristiques des cellules hématopoïétiques (CD45) ou des monocytes/macrophages (CD 14), ni les marqueurs CD 144 et CD31, caractéristiques des cellules endothéliales différenciées (Figure 2c). Ces résultats montrent que l'expansion cellulaire pendant 1-3 jours *in vitro* (ou *ex vivo*) favorise la prolifération d'une population homogène de cellules (cellules FSV-CULT), possédant quelques antigènes de surface caractéristiques des cellules is potentiel pro-angiogénique mais aucun marqueur de surface caractéristique de cellules différenciées

### EXEMPLE 3 : Induction de la néovascularisation dans le muscle ischémié de souris, par des cellules FSV-CULT et différenciation de cette population en cellules endothéliales.

### 3.1 Matériel et méthodes

Des souris mâles nu/nu (Harlan, France) âgées de 7 semaines sont élevées dans les mêmes conditions que celles exposées dans l'Exemple 1.

Les échantillons de tissu adipeux humains sont identiques à ceux utilisés dans l'Exemple 2.

Les cellules humaines et de souris FSV-CULT sont isolées comme précisé dans les Exemples 1 et 2.

La quantification de la néovascularisation et l'analyse phénotypique sont réalisées comme précisé, respectivement dans les Exemples 1 et 2.

### 3.2 Résultats

L'effet de l'injection (ou greffe) des cellules FSV-CULT humaines sur la revascularisation est évalué chez des souris immuno-déficientes *Nude.* Comme pour les cellules FSV-CULT de souris, l'injection de 1×10⁶ cellules FSV-CULT humaines après 15 jours d'ischémie des membres inférieurs, permet d'obtenir une augmentation significative du score angiographique ainsi que du flux sanguin cutané (facteur, respectivement 1,6 et 1,5, lorsqu'on compare aux souris *Nude* ischémiées non-traitées, P<0,01) (Figures 3a et 3b). Deux mécanismes possibles, qui ne s'excluent pas, peuvent expliquer les effets pro-angiogéniques : la libération de facteurs de croissance angiogéniques par les cellules FSV-CULT ou une contribution directe des cellules injectées par incorporation (ou greffe) de celles-ci dans les vaisseaux régénérés. En effet, le VEGF est détecté comme étant un facteur angiogénique potentiel (31±8 ng/ml).

Ainsi, afin d'évaluer la capacité des cellules FSV-CULT à être incorporées dans de nouveaux vaisseaux sanguins, des expériences immunochimiques ont été réalisées en utilisant un anticorps spécifique du marqueur CD31 humain, qui ne réagit pas avec du tissu de souris. De nombreuses cellules positives pour le marqueur CD31 tapissant les vaisseaux régénérés sont mises en évidence dans le membre inférieur traité (Figure 3c). Aucune cellule positive pour le marqueur CD31 n'est détectée dans l'autre membre inférieur non-traité. La détection de cellules CD31+ humaines suggère fortement que, dans des conditions *in vivo,* les cellules FSV-CULT se différencient en cellules endothéliales et contribuent directement à la régénération des vaisseaux.

### EXEMPLE 4 : Différenciation spontanée des cellules FSV-CULT humaines en adipocytes ou en cellules endothéliales, in vitro et in vivo dans la matrice de Matrigel^{®}.

### 4.1 Matériel et méthodes

Les cellules humaines de la fraction stromale vasculaire extramédullaire (FSV) sont préparées et mises en culture comme dans l'Exemple 2.

Pour tester leur potentiel de différenciation *in vitro* au niveau clonal en préservant la fonction cellulaire, les cellules FSV-CULT sont mises en culture dans du milieu semi-solide (méthyl-cellulose; 15), Une culture primaire de cellules FSV-CULT est trypsinée, puis ensemencés à une concentration de 7×10³ cellules/ml dans 1,5 ml de Methocult MG3534, MG, H4534 (StemCell Technologies) ou tout autre milieu équivalent. Les cellules sont cultivées pendant 10 jours pour stimuler leur développement en cellules ayant une morphologie de type endothélial, puis analysées par immunomarquage. Les colonies des cultures en présence de méthyl-cellulose sont lavées avec du tampon PBS et fixées dans un mélange de méthanol/acétone pendant 20 minutes à -20°C. Les préparations sont alors bloquées dans du PBS contenant 1 % de BSA, et incubées pendant 1 heure avec soit des anticorps anti-CD3 humains (Dako, référence MO823) ou des anticorps anti-facteur vWF humain ou de souris.

Le test d'angiogénèse, *in vivo,* à l'aide de la matrice de Matrigel^{®} est réalisé de la façon suivante. Les souris reçoivent une injection, par voie sous-cutanée, d'un volume de 0,5 ml de matrice de Matrigel^{®} contenant 10⁶ de cellules FSV-CULT isolées à partir de tissu de souris, ou de tissu humain. Au 14^{ième} jour, les souris sont sacrifiées et l'angiogénèse est analysée comme décrit dans Tamarat R et al. (37). Pour l'immunomarquage, les matrices de Matrigel^{®} sont traités comme décrit dans Nibbelink M. et al. (35). Des sections de 5 µm d'épaisseur sont colorées par la phosphatase alcaline (BCIP/NBT) après avoir été incubées avec un anticorps de Jackson couplés à la phosphatase alcaline, ou alors elles sont colorées par la diaminobenzidine (DAB) après avoir été incubées avec un anticorps primaire puis par un anticorps secondaire biotinylé (Dako Carpinteria, CA) ; l'anticorps anti-complexe IV OxPhos humain provient de Molecular Probes (Eugene, Orégon, USA).

A titre de comparaison, des cellules FSV-CULT sont cultivées dans un milieu adipogénique (Björntorp et al., précité).

### _{4.2 Résultats}

La différenciation des cellules FSV-CULT a été analysée *in vitro,* dans un milieu semi-solide permettant d'étudier la différenciation cellulaire au niveau clonal en préservant la fonction des cellules (méthyl-cellulose), et *in vivo* après injection de cellules associées à une matrice solide (Matrigel^{®})

Dans ces conditions, les cellules FSV-CULT forment un réseau ayant une structure en forme de tubes creux (Figure 4b). Des anticorps dirigés respectivement contre le marqueur CD31 et contre le facteur de von Willebrand (vWF) marquent fortement les cellules FSV-CULT (Figures 4c et d). Lorsque les cellules FSV-CULT sont injectées en association avec une matrice de Matrigel^{®}, les cellules forment de nombreuses structures de type tubulaire à l'intérieur de la matrice de Matrigel^{®}. La présence d'érythrocytes dans le lumen de ces structures de type tubulaire démontre l'existence d'une structure vasculaire fonctionnelle (Figure 4e et f). Les anticorps dirigés contre le marqueur CD31 et contre le marqueur vWF marquent positivement ces structures ressemblant à des vaisseaux (Figure 4g et h).

Par comparaison, les cellules FSV-CULT cultivées dans un milieu adipogénique se différencient en adipocytes (figure 4a).

L'ensemble de ces résultats montre que les cellules FSV-CULT présentent spontanément les propriétés phénotypiques et fonctionnelles de cellules progénitrices endothéliales.

### EXEMPLE 5 : Dédifférenciation d'adipocytes humains matures en culture.

### 5.1 Matériel et méthodes

### - Dédifférenciation d'adipocytes humains matures

La fraction d'adipocytes humains matures, isolée à partir d'un échantillon de tissu adipeux comme décrit à l'exemple 1, est lavée délicatement dans du milieu DMEM F12 supplémenté par 10% de SVN et préparée sous forme de suspension à une concentration de 10⁶ cellules/ml. Un échantillon de 100 µl de la suspension cellulaire est transféré sur une lamelle couvre-objet Thermanox de 25 mm et placé dans une boite de culture de 35 mm. La première lamelle est recouverte par une seconde, et après une incubation pendant 15 minutes à température ambiante, on ajoute 1,5 ml de DMEM F12 supplémenté par 10% de SVN. Après 4 ou 5 jours d'incubation, les cellules adhérentes contenant des petites gouttelettes lipidiques (cellules de type pré-adipocyte) apparaissent ; elles se modifient en une morphologie de type fibroblaste qui sont dépourvues de gouttelettes de lipides (cellules hDDAC pour *human dedifferenciated adipose cells*).

Ces cellules de type fibroblastique entrent alors en division active et peuvent subir quelques passages sans modification majeure de leurs caractéristiques.

Les adipocytes humains dédifférenciés sont mis en culture en méthyl-cellulose et analysés par immunomarquage comme décrit à l'exemple 4. En outre, leur potentiel angiogénique est analysé *in vivo,* après injection dans une matrice de Matrigel^{®}, comme décrit à l'exemple 4. Le potentiel angiogénique des cellules FSV-CULT préparées comme décrit à l'exemple 3 est analysé en parallèle.

Alternativement, les adipocytes humains dédifférenciés sont mis en culture dans du milieu adipogénique (Björntorp et al., précité).

### 5.2 Résultats

Afin d'obtenir une population homogène de cellules précurseurs d'adipocytes à partir de tissu adipeux et de confirmer l'existence d'un précurseur commun aux adipocytes et aux cellules endothéliales dérivés des cellules FSV-CULT, des adipocytes matures ont été dédifférenciés, selon des protocoles précédemment décrits (16 ; 17 ; 18 ; 19). Les adipocytes matures isolés à partir du tissu adipeux représentent 99% d'une population de cellules flottantes. La seule contamination cellulaire provient de macrophages riches en gouttelettes lipidiques, avec un rapport de quelques cellules contaminantes pour 1000 cellules. Lorsque les adipocytes sont mis en culture dans les conditions précitées (17), ils perdent initialement leurs acides gras et changent leur morphologie en cellules de type pré-adipocyte puis en cellules de type fibroblastique qui peuvent se fixer à la lamelle. Ce changement morphologique est associé à des changements fonctionnels étant donné que les adipocytes perdent également leur contenu enzymatique pour la lipolyse et la lipogénèse ainsi que les marqueurs moléculaires (17).

La population homogène d'adipocytes humains dédifférenciés (hDDAC) a la capacité de proliférer et de se différencier à nouveau en adipocytes, lorsqu'elle est cultivée en milieu adipogénique (Figure 5a).

La même population homogène d'adipocytes humains dédifférenciés (hDDAC) cultivée dans un milieu contenant de la méthyl-cellulose, forme des alignements branchus et des structures en forme de tube (Figure 5b) et co-exprime, à plus de 99%, les mêmes marqueurs que les cellules FSV-CULT (CD13, CD34 et HLA ABC), incluant le marqueur vWF (Figure 5c).

Comme c'est le cas pour les cellules FSV-CULT, lorsque les cellules hDDAC sont injectées en association avec la matrice de Matrigel^{®}, elles forment de nombreuses structures de type tubulaire, qui contiennent des érythrocytes dans leur lumen démontrant l'existence d'une structure vasculaire fonctionnelle.

Ces résultats sont illustrés dans la figure 6 qui illustre la plasticité des cellules de la lignée adipocytaire, pour l'obtention de cellules endothéliales. Les cellules progénitrices adipocytaires ont la capacité de se différencier en adipocytes et d'acquérir un phénotype endothélial fonctionnel. Les adipocytes matures peuvent se dédifférencier en cellules progénitrices à double potentiel prolifératif.

### EXEMPLE 6 : Stimulation de la néovascularisation dans le muscle ischémié de souris, par des adipocytes humains dédifférenciés souris et différenciation de ces adipocytes en cellules endothéliales.

### 6.1 Matériel et méthodes

Le potentiel angiogénique des cellules hDDAC a été analysé chez la chez les souris *Nude* comme pour les cellules FSV-CULT (exemple 3) qui servent de comparaison.

### 6.2 Résultats

Les cellules hDDAC sont aussi efficaces que les cellules FSV-CULT pour restaurer la vascularisation des membres inférieurs ischémiés (Figures 5d et 5e). Comme c'est le cas pour les cellules FSV-CULT, on identifie de nombreuses cellules positives pour le marqueur CD31 qui tapissent les vaisseaux nouvellement formés du membre inférieur, dans lequel les cellules hDDAC ont été injectées (Figure 5f).

### EXEMPLE 7: Utilisation des cellules FSV-CULT pour induire une néovascularisation dans un contexte athéromateux (modèle murin ApoE -/-).

### 7.1) Matériels et méthodes

Le potentiel angiogénique des cellules FSV-CULT a été analysé chez des souris déficientes en ApoE (ApoE *Knock-out* (ApoE KO ou ApoE -/-) ; Iffa-Credo), âgées de 14 semaines, comme chez la souris C57B1/6 (exemple 1). Le potentiel angiogénique des cellules mononucléées de la moelle osseuse, chez des souris ApoE KO, est analysé en parallèle, à titre de comparaison. Le groupe contrôle reçoit une injection de PBS, dans les mêmes conditions.

De manière plus, précise, le processus de néovascularisation a été analysé par microangiographie et laser doppler, 4 semaines après l'occlusion fémorale. L'analyse statistique a été effectuée par un test de variance de type ANOVA pour comparer chaque paramètre (n = 6 pour chaque groupe). Un test t de Bonferroni a ensuite permis d'identifier les groupes à l'origine de ces différences. Une valeur de P <0,05 est considérée comme significative.

### 7.2) Résultats

L'administration de cellules FSV-CULT augmente le score angiographique d'un facteur 2 (p<0,01) et le flux sanguin d'un facteur 1,5 (p<0,01), dans le membre inférieur ischémie des souris ApoE KO traitées, par rapport aux souris ApoE KO non traitées (Tableau I). Le potentiel angiogénique des cellules FSV-CULT adipeux est similaire à celui des cellules mononucléées de la moelle osseuse (Tableau I).

**Tableau I : Potentiel angiogénique des cellules FSV-CULT chez les souris ApoE -/-**

| **Traitement** | **Score angiographique* (membre ischémié/ membre non-ischémié)** | **Flux sanguin cutané* (membre ischémié/ membre non-ischémié)** |
|---|---|---|
| **FSV-CULT** | 0,982 ± 0,3 | 0,963 ± 0,04 |
| **BM-MNC** | 1,002 ± 0,04 | 0.995 ± 0,03 |
| **PBS** | 0,47 ± 0,02 | 0,65 ± 0,03 |

| | | |
|---|---|---|
| ***Les valeurs représentent la moyenne ± écart-type sur un groupe de 6 animaux** | | |

Le traitement du membre ischémié des souris ApoE (-/-) est efficace et favorise l'angiogenèse/néovascularisation. Cet effet est aussi efficace que l'injection de cellules mononuclées de la moelle osseuse. Les cellules FSV-CULT conservent leur potentiel pro-angiogénique dans un contexte athéromateux.

### EXEMPLE 8 : Amélioration du potentiel angiogénique des cellules FSV-CULT par modification de leur état redox.

### 8.1) Matériels et méthodes

Le potentiel angiogénique des cellules FSV-CULT traitées, *in vitro* avec de l'antimycine (40nM) et/ou de la pyrrolidine dithiocarbamate (PDTC ; 0,5 mM) deux jours avant l'injection, a été analysé dans le modèle de la souris avec un membre inférieur ischémié comme décrit à l'exemple 1. En outre, après l'injection des cellules FSV-CULT traitées avec l'antimycine seule, par addition d'antimycine dans le milieu de culture, ou non-traitées, les souris ont reçu ou non, une injection quotidienne i.p. d'antimycine (50 µL à 40nM). Les souris traitées, de façon similaire avec le PDTC seul ou en association avec l'antimycine, ne reçoivent aucun traitement après l'injection cellulaire.

Le potentiel angiogénique des cellules FSV-CULT non-traitées, chez des souris non-traitées après l'injection des cellules, a été analysé en parallèle, à titre de comparaison. Le groupe contrôle a reçu une injection d'éthanol, dans les mêmes conditions.

Le processus de néovascularisation a été analysé par microangiographie et éventuellement par laser Doppler, 8 jours après l'occlusion fémorale. L'analyse statistique a été effectuée par un test de variance de type ANOVA pour comparer chaque paramètre (n = 5 pour chaque groupe). Un test t de Bonferroni a ensuite permis d'identifier les groupes à l'origine de ces différences. Une valeur de P <0,05 est considérée comme significative.

### 8.2) Résultats

L'effet de la modification de l'état redox des cellules FSV-CULT sur leur potentiel pro-angiogénique, a été testé avec un inhibiteur du complexe III de la chaîne respiratoire mitochondriale qui induit la production d'espèces actives de l'oxygène et une modification de l'état redox des cellules (antimycine), et d'un agent antioxydant qui limite la production d'espèces actives de l'oxygène et l'état redox cellulaire (PDTC : pyrrolidine dithiocarbamate). Les résultats sont présentés dans les tableaux II et III ci-après.

**Tableau II : Effet du traitement in vitro ou in vivo, des cellules FSV-CULT par l'antimycine, sur le potentiel angiogénique des cellules**

| **Injection** | **Flux sanguin dans le membre ischémié/membre non-ischémié** |
|---|---|
| **éthanol** | 0,430 ± 0,025 |
| **Cellules FSV-CULT** | 0,616 ± 0,062 |
| **Cellules FSV-CULT traitées avec l'antimycine puis antimycine (i.p.)** | 0,830 ± 0,031 |
| **Cellules FSV-CULT non-traitées puis antimycine (i.p.)** | 0,426 ± 0,022 |

**Tableau III : Effet du traitement, in vitro, des cellules FSV-CULT par l'antimycine et/ou le PDTC sur le potentiel angiogénique des cellules**

| **Injection** | **Flux sanguin dans le membre ischémié/membre non-ischémié** | **Score angiographique dans le membre ischémie/membre non-ischémié** |
|---|---|---|
| **éthanol** | 0,430 ± 0,025 | 0,588 ± 0,033 |
| **Cellules FSV-CULT** | 0,690 ± 0,014 | 0,844 ± 0,027 |
| **Cellules FSV-CULT traitées avec l'antimycine** | 0,882 ± 0,015 | 1,094 ± 0,030 |
| **Cellules FSV-CULT traitées avec l'antimycine et le PDTC** | 0,716 ± 0,024 | 0.846 ± 0,026 |
| **Cellules FSV-CULT traitées avec le PDTC** | 0,718 ± 0,041 | 0,774 ± 0,023 |

Le traitement des cellules FSV-CULT avec l'antimycine seule, avant l'injection dans le membre ischémié, a un effet significativement positif et important sur la revascularisation, comme le montre l'augmentation d'un facteur 1,4, du flux sanguin (p<0.05 ; Tableaux II et III) et d'un facteur 1,3 du score angiographique (p=0.06 ; Tableau IIII). Cet effet est prévenu par un antioxydant (Tableau III), ce qui indique l'implication des espèces actives de l'oxygène et/ou une modification de l'état redox dans les effets favorables à l'angiogenèse. En revanche, l'antimycine n'a pas d'effet lorsqu'elle est administrée directement chez l'animal, après l'injection des cellules FSV-CULT dans le muscle ischémie (Tableau II).

### RÉFÉRENCES BIBLIOGRAPHIQUES

1. Ailhaud G. et al., Annu. Rev. Nutr., 1992, 12, 207-33.
2. Castellot J.J. et al., Proc. Natl. Acad. Sci. USA, 1982, 79, 5597-601.
3. Bouloumie A. et al., Ann. Endocrino/. (Paris), 2002, 63, 91-5.
4. Wasserman P., The development of adipose tissue, 1965.
5. Dobson D.E. et al., Cell, 1990, 61, 223-30.
6. Claffey KP. et al., J. Biol. Chem., 1992, 267, 16316-22.
7. Bouloumie A. et al., Circ. Res., 1998, 83, 1059-66.
8. Sierra-Honigmann M.R. et al., Science, 1998, 281, 1683-6.
9. Rupnick M.A. et al., Proc. Natl. Acad. Sci. USA, 2002, 99, 10730-5.
10. Zuk PA. et al., Mol. Biol. Cell, 2002, 13, 4279-95.
11. Erickson GR. et al., Biochem. Biophys. Res. Commun., 2002, 290, 763-9.
12. Cousin B. et al., Biochem. Biophys. Res. Commun., 2003, 301, 1016-22.
13. Safford KM. et al., Biochem. Biophys. Res. Commun., 2002, 294, 371-9.
14. Bjorntorp P. et al., J. Lipid Res., 1978, 19, 316-24.
15. Gehling U.M. et al., Blood, 2000, 95, 3106-12.
16. Negrel R. et al., Proc. Natl. Acad. Sci USA, 1978, 75, 6054-8.
17. Negrel R. et al., Methods Enzymol., 1985, 109, 377-385.
18. Aoki S. et al., Cell Struct. Funct., 2003, 28, 55-60.
19. Shigematsu M. et al., Cell. Struct. Funct., 1999, 24, 89-100.
20. Demandez Pujol B. et al., Differentiation, 2000, 65, 287-300.
21. Rehman J. et al., Circulation, 2003, 107, 1164-9.
22. JiangY. et al., Nature, 2002, 418, 41-9.
23. Jiang Y. et al., Exp. Hematol., 2002, 30, 896-904.
24. Gronthos S. et al., J. Cell Physiol., 2001,189, 54-63.
25.Carmeliet P., Nat. Med., 2003, 9, 653-60.
26. Orlic D. et al., Nature, 2001, 410, 701-705.
27. Kocher AA. et al., Nat. Med., 2001, 7, 430-436.
28. Assmus B. et al., Circulation, 2002,106, 3009-17.
29. Strauer B.E. et al., Circulation, 2002,106, 1913-8.
30. Tateishi-Yuyama E. et al., Lancet, 2002, 360, 427-35.
31. Tse H.F. et al., Lancet, 2003, 361, 47-9.
32. Stamm C. et al., Lancet, 2003, 361, 45-6.
33. Charrière G. et al., J. Biol. Chem., 2003, 278, 9850-5.
34. Mallat Z. et al., Circ. Res., 2002, 91, 441-8.
35. Nibbelink M. et al., J. Biol. Chem., 2001, 276, 47291-5.
36. Silvestre J.S. et al., Circ. Res., 2001, 89, 259-64.
37. Tamarat R. et al., Lab. Invest., 2002, 82, 747-56.
38. Luttun A. et al., Trends Cardiovasc. Med., 2002,12, 88-96.
39. Iba O.et al., Circulation, 2002, 106, 2019-2025.
40. Benelli R et al., Internat. J. Biol. Markers, 1999,14, 4, 243-246.

## Revendications

1. Utilisation de cellules du tissu adipeux blanc, médullaire ou extramédullaire formant des sous-populations homogènes, CD13⁺, HLA ABC⁺, CD14⁻, CD45⁻, CD31⁻, CD 144⁻ et CD34⁺, pour la préparation d'un médicament destiné à la reconstruction totale ou partielle d'un réseau vasculaire fonctionnel.

2. Utilisation selon la revendication 1, **caractérisée en ce que** lesdites cellules du tissu adipeux sont représentées par une sous-population homogène de cellules de la fraction stromale vasculaire extra-médullaire, susceptibles d'être obtenues par expansion cellulaire limitée en culture.

3. Utilisation selon la revendication 2, **caractérisée en ce que** ladite sous-population homogène de cellules de la fraction stromale vasculaire extra-médullaire est susceptible d'être obtenue par une expansion cellulaire limitée par un nombre de passages successifs inférieur à 10 desdites cellules.

4. Utilisation selon la revendication 1, **caractérisée en ce que** lesdites cellules du tissu adipeux sont représentées par une sous-population homogène d'adipocytes matures dédifférenciés.

5. Utilisation selon l'une quelconque des revendications 1 à 4, **caractérisée en ce que** lesdites cellules du tissu adipeux formant des sous-populations homogènes, exprimant au moins les antigènes de surface CD 13 et HLA ABC, sont associées à un support polymérique solide ou semi-solide.

6. Utilisation selon la revendication 5, **caractérisée en ce que** ledit support polymérique solide est sélectionné dans le groupe constitué par les matrices membranaires basales reconstituées comprenant au moins l'un des éléments suivants : collagène, laminine et protéoglycanes, et les matrices extracellulaires reconstituées comprenant l'un des éléments suivants : fibronectine, collagène, laminine et thrombospondine.

7. Utilisation selon la revendication 5 ou la revendication 6, **caractérisé en ce que** ledit support polymérique peut en outre comprendre des enzymes de dégradation desdites matrices ainsi que des inhibiteurs enzymatiques et des facteurs de croissance.

8. Utilisation selon la revendication 5, **caractérisée en ce que** ledit support polymérique semi-solide est de préférence un dérivé cellulosique et notamment de la méthyl-cellulose.

9. Utilisation selon l'une quelconque des revendications 1 à 8, **caractérisée en ce que** lesdites cellules sont génétiquement modifiées.

10. Utilisation selon la revendication 9, **caractérisée en ce que** lesdites cellules comprennent au moins une mutation d'un gène autologue.

11. Utilisation selon la revendication 9, **caractérisée en ce que** lesdites cellules contiennent au moins une copie d'un gène hétérologue.

12. Utilisation selon l'une quelconque des revendications 9 à 11, **caractérisée en ce que** lesdites cellules sont d'origine humaine.

13. Utilisation d'une composition contenant des cellules du tissu adipeux blanc, médullaire ou extra-médullaire formant des sous-populations homogènes, exprimant au moins les antigènes de surface CD13 et HLA ABC telles que définies l'une quelconque des revendications 1 à 12, et au moins un véhicule et/ou un support approprié à une administration parentérale ou intrasite, pour la préparation d'un médicament destiné à la reconstruction totale ou partielle d'un réseau vasculaire fonctionnel.

14. Composition pharmaceutique contenant des cellules du tissu adipeux blanc, médullaire ou extra-médullaire formant des sous-populations homogènes, exprimant au moins les antigènes de surface CD 13 et HLA ABC et n'exprimant pas les antigènes de surface CD 14, CD45, CD31 et CD 144, telles que définies à l'une quelconque des revendications 1 à 4 et 9 à 12, lesdites cellules étant associées à un support polymérique solide ou semi-solide tel que défini à l'une quelconque des revendications 5 à 8, et au moins un véhicule et/ou un support approprié à une administration parentérale ou intrasite.

15. Procédé de culture de cellules du tissu adipeux blanc médullaire ou extra-médullaire formant des sous-populations homogènes, exprimant au moins les antigènes de surface CD13 et HLA ABC, lequel procédé est **caractérisé en ce qu'**il comprend au moins les étapes suivantes :
- expansion cellulaire limitée de cellules de la fraction stromale vasculaire extra-médullaire ou d'adipocytes matures dédifférenciés, par un nombre de passages successifs inférieurs à 10 dites cellules, sur un support de culture solide convenable, dans un milieu comprenant au moins un facteur de croissance apte à stimuler la formation de cellules endothéliales et éventuellement au moins une cytokine convenable ;
- modification, de manière continue ou transitoire, de l'environnement en oxygène de la culture et
- modification de manière continue ou transitoire, de l'équilibre redox desdites cellules ou de la production d'espèces actives de l'oxygène par lesdites cellules par ajout de molécules pro- ou antioxydantes dans le milieu extra ou intracellulaire.

16. Procédé selon la revendication 15, **caractérisé en ce que** le facteur de croissance apte à stimuler la formation de cellules endothéliales est notamment du VEGF, de préférence à une concentration d'environ 10 ng/ml.

17. Procédé selon la revendication 15 ou la revendication 16, **caractérisé en ce que** l'environnement en oxygène de la culture est à 1 % ; de quelques heures à quelques jours.

18. Procédé de criblage de molécules actives sur les cellules endothéliales différenciées, lequel procédé est **caractérisé en ce qu'**il comprend au moins les étapes suivantes :
- la culture de cellules du tissu adipeux blanc médullaire ou extra-médullaire formant des sous-populations homogènes, exprimant au moins les antigènes de surface CD13 et HLA ABC et n'exprimant pas les antigènes de surface CD14, CD45, CD31 et CD144, telles que définies à l'une quelconque des revendications 1 à 4 et 9 à 12, dans un milieu de culture polymérique semi-solide,
- la mise en contact des cellules endothéliales différenciées ainsi obtenues, avec une banque de molécules à tester, et
- l'identification et la sélection des molécules actives sur lesdites cellules endothéliales différenciées.

## Claims

1. Use of cells of medullary or extramedullary white adipose tissue forming homogeneous CD13⁺, HLA ABC⁺, CD14⁻, CD45⁻, CD31⁻, CD144⁻ and CD34⁺ sub-populations, for the preparation of a medicament for the total or partial reconstruction of a functional vascular network.

2. Use according to claim 1, **characterised in that** the cells of the adipose tissue are represented by a homogeneous sub-population of cells of the extramedullary stromal vascular fraction which are capable of being obtained by limited cell expansion in culture.

3. Use according to claim 2, **characterised in that** the homogeneous sub-population of cells of the extramedullary stromal vascular fraction is capable of being obtained by limited cell expansion by a number of successive passages of the said cells of less than 10.

4. Use according to claim 1, **characterised in that** the cells of the adipose tissue are represented by a homogeneous sub-population of dedifferentiated mature adipocytes.

5. Use according to any one of claims 1 to 4, **characterised in that** the cells of the adipose tissue forming homogeneous sub-populations, expressing at least the CD13 and HLA ABC surface antigens, are associated with a solid or semi-solid polymer support.

6. Use according to claim 5, **characterised in that** the solid polymer support is selected from the group composed of reconstituted basement membrane matrices comprising at least one of the following elements: collagen, laminin and proteoglycans, and reconstituted extracellular matrices comprising one of the following elements: fibronectin, collagen, laminin and thrombospondin.

7. Use according to claim 5 or claim 6, **characterised in that** the polymer support may also comprise degradation enzymes of the matrices and also enzyme inhibitors and growth factors.

8. Use according to claim 5, **characterised in that** the semi-solid polymer support is preferably a cellulose derivative and especially methylcellulose.

9. Use according to any one of claims 1 to 8, **characterised in that** the cells are genetically modified.

10. Use according to claim 9, **characterised in that** the cells comprise at least one mutation of an autologous gene.

11. Use according to claim 9, **characterised in that** the cells contain at least one copy of a heterologous gene.

12. Use according to any one of claims 9 to 11, **characterised in that** the cells are of human origin.

13. Use of a composition containing cells of medullary or extramedullary white adipose tissue forming homogeneous sub-populations, expressing at least the CD13 and HLA ABC surface antigens as defined in any one of claims 1 to 12, and at least one vehicle and/or one support suitable for parenteral or intrasite administration, for the preparation of a medicament for the total or partial reconstruction of a functional vascular network.

14. Pharmaceutical composition containing cells of medullary or extramedullary white adipose tissue forming homogeneous sub-populations, expressing at least the CD13 and HLA ABC surface antigens and not expressing the CD14, CD45, CD31 and CD144 surface antigens, as defined in any one of claims 1 to 4 and 9 to 12, the cells being associated with a solid or semi-solid polymer support as defined in any one of claims 5 to 8, and at least one vehicle and/or one support suitable for parenteral or intrasite administration.

15. Method for culturing cells of medullary or extramedullary white adipose tissue forming homogeneous sub-populations, expressing at least the CD13 and HLA ABC surface antigens, which method is **characterised in that** it comprises at least the following steps:
- limited cell expansion of cells of the extramedullary stromal vascular fraction or of dedifferentiated mature adipocytes, by a number of successive passages of the said cells of less than 10, on an appropriate solid culture support, in a medium comprising at least one growth factor suitable for stimulating the formation of endothelial cells and optionally at least one appropriate cytokine;
- continuous or transitory modification of the oxygen environment of the culture and
- continuous or transitory modification of the redox equilibrium of the cells or of the production of active oxygen species by the cells by the addition of pro- or antioxidant molecules to the extracellular or intracellular medium.

16. Method according to claim 15, **characterised in that** the growth factor suitable for stimulating the formation of endothelial cells is especially VEGF, preferably at a concentration of approximately 10 ng/ml.

17. Method according to claim 15 or claim 16, **characterised in that** the oxygen environment of the culture is at 1%; from a few hours to a few days.

18. Method for screening molecules active on differentiated endothelial cells, which method is **characterised in that** it comprises at least the following steps:
- culturing cells of medullary or extramedullary white adipose tissue forming homogeneous sub-populations, expressing at least the CD13 and HLA ABC surface antigens and not expressing the CD14, CD45, CD31 and CD144 surface antigens, as defined in any one of claims 1 to 4 and 9 to 12, in a semi-solid polymer culture medium,
- bringing the differentiated endothelial cells so obtained into contact with a bank of molecules to be tested, and
- identifying and selecting the molecules active on the differentiated endothelial cells.

## Patentansprüche

1. Verwendung von Zellen des medullären oder extramedullären weißen Fettgewebes, die homogene Subpopulationen bilden, nämlich CD13⁺, HLA ABC⁺, CD14⁻, CD45⁻, CD31⁻, CD144⁻ und CD34⁺, zur Herstellung eines Medikaments, das für eine vollständige oder partielle Rekonstruktion eines funktionellen Vaskulärsystems bestimmt ist.

2. Verwendung nach Anspruch 1, **dadurch gekennzeichnet, dass** die Zellen des Fettgewebes von einer homogenen Subpopulation von Zellen der extramedullären stromavaskulären Fraktion dargestellt sind, die durch limitierte Zellexpansion in Kultur erhalten werden können.

3. Verwendung nach Anspruch 2, **dadurch gekennzeichnet, dass** die homogene Subpopulation von Zellen der extramedullären stromavaskulären Fraktion durch eine limitierte Zellexpansion mit weniger als 10 aufeinanderfolgenden Passagen der Zellen erhalten werden kann.

4. Verwendung nach Anspruch 1, **dadurch gekennzeichnet, dass** die Zellen des Fettgewebes von einer homogenen Subpopulation von reifen dedifferenzierten Adipozyten dargestellt werden.

5. Verwendung nach einem der Ansprüche 1 bis 4, **dadurch gekennzeichnet, dass** die Zellen des Fettgewebes, die homogene Subpopulationen bilden und die wenigstens die Oberflächenantigene CD13 und HLA ABC exprimieren, an einem festen oder halbfesten Polymerträger haften.

6. Verwendung nach Anspruch 5, **dadurch gekennzeichnet, dass** der feste Polymerträger ausgewählt ist aus der Gruppe bestehend aus rekonstituierten basalen Membranmatrizen, umfassend wenigstens eines der folgenden Elemente: Kollagen, Laminin und Proteoglykane, und rekonstituierten extrazellulären Matrizen, umfassend eines der folgenden Elemente: Fibronectin, Kollagen, Laminin und Thrombospondin.

7. Verwendung nach Anspruch 5 oder Anspruch 6, **dadurch gekennzeichnet, dass** der Polymerträger weiterhin Enzyme zum Abbau der Matrizen, sowie enzymatische Inhibitoren und Wachstumspromotoren umfassen kann.

8. Verwendung nach Anspruch 5, **dadurch gekennzeichnet, dass** der halbfeste Träger bevorzugt ein Zellulosederivat, insbesondere Methylzellulose, ist.

9. Verwendung nach einem der Ansprüche 1 bis 8, **dadurch gekennzeichnet, dass** die Zellen gentechnisch modifiziert sind.

10. Verwendung nach Anspruch 9, **dadurch gekennzeichnet, dass** die Zellen wenigstens eine Mutation eines autologen Gens umfassen.

11. Verwendung nach Anspruch 9, **dadurch gekennzeichnet, dass** die Zellen wenigstens eine Kopie eines heterologen Gens enthalten.

12. Verwendung nach einem der Ansprüche 9 bis 11, **dadurch gekennzeichnet, dass** die Zellen menschlichen Ursprungs sind.

13. Verwendung einer Zusammensetzung, enthaltend Zellen des medullären oder extramedullären weißen Fettgewebes, die homogene Subpopulationen bilden und die wenigstens die Oberflächenantigene CD13 und HLA ABC exprimieren, nach einem der Ansprüche 1 bis 12, und wenigstens ein Vehikel und/oder einen Träger, die für eine parenterale oder intra-site Verabreichung geeignet sind, zur Herstellung eines Medikaments, das für eine vollständige oder partielle Rekonstruktion eines funktionellen Vaskulärsystems bestimmt ist.

14. Pharmazeutische Zusammensetzung, enthaltend Zellen des medullären oder extramedullären weißen Fettgewebes, die homogene Subpopulationen bilden und die wenigstens die Oberflächenantigene CD13 und HLA ABC exprimieren und die nicht die Oberflächenantigene CD14, CD45, CD31 und CD144 exprimieren, nach einem der Ansprüche 1 bis 4 und 9 bis 12, wobei die Zellen auf einem festen oder halbfesten Träger nach einem der Ansprüche 5 bis 8 haften, und wenigstens ein Vehikel und/oder einen Träger, die für eine parenterale oder intra-site Verabreichung geeignet sind.

15. Verfahren zur Kultivierung von Zellen des medullären oder extramedullären weißen Fettgewebes, die homogene Subpopulationen bilden und die wenigstens eines der Oberflächenantigene CD13 und HLA ABC exprimieren, wobei das Verfahren **dadurch gekennzeichnet ist, dass** es wenigstens folgende Schritte umfasst:
- limitierte Zellexpansion von Zellen der extramedullären stromavaskulären Fraktion oder von reifen dedifferenzierten Adipozyten mit weniger als 10 aufeinanderfolgenden Passagen der Zellen auf einem geeigneten festen Kulturträger in einer Umgebung umfassend wenigstens einen Wachstumsfaktor, der in der Lage ist, die Bildung von Endothelzellen zu stimulieren, und gegebenenfalls ein geeignetes Zytokin;
- kontinuierliche oder transiente Veränderung der Sauerstoffumgebung der Kultur, und
- kontinuierliche oder transiente Veränderung des Redoxgleichgewichts der Zellen oder der Produktion von aktiven Sauerstoffspezies durch die Zellen durch Zugabe von oxidierenden oder antioxidierenden Molekülen in das extra-oder intrazelluläre Milieu.

16. Verfahren nach Anspruch 15, **dadurch gekennzeichnet, dass** der Wachstumsfaktor, der in der Lage ist, die Bildung von Endothelzellen zu stimulieren, insbesondere VEGF ist, bevorzugt bei einer Konzentration von etwa 10 ng/ml.

17. Verfahren nach Anspruch 15 oder Anspruch 16, **dadurch gekennzeichnet, dass** die Sauerstoffumgebung der Kultur für einige Stunden bis einige Tage 1% ist.

18. Verfahren zum Screenen von aktiven Molekülen auf den differenzierten Endothelzellen, wobei das Verfahren **dadurch gekennzeichnet ist, dass** es wenigstens die folgenden Schritte umfasst:
- Kultivieren von Zellen des medullären oder extramedullären weißen Fettgewebes, die homogene Subpopulationen bilden und die wenigstens die Oberflächenantigene CD13 und HLA ABC exprimieren und die nicht die Oberflächenantigene CD14, CD45, CD31 und CD144 exprimieren, nach einem der Ansprüche 1 bis 4 und 9 bis 12 in einer halbfesten polymeren Kulturumgebung,
- In-Kontakt-bringen der auf diese Art erhaltenen differenzierten Endothelzellen mit einer Molekülbibliothek zum Testen, und
- Identifizierung und Selektion von aktiven Molekülen auf den differenzierten Endothelzellen.
